# EUROPÄISCHE PATENTSCHRIFT

(11) **EP 0 765 335 B1**
(45) Veröffentlichungstag und Bekanntmachung des Hinweises auf die Patenterteilung: **01.09.1999**
(21) Anmeldenummer: 95921702.7
(22) Anmeldetag: 14.06.1995
(51) Int. Cl.: C07H 1/08, C07H 21/00, C12N 15/10

(54) **UNIVERSELLES VERFAHREN ZUR ISOLIERUNG UND REINIGUNG VON NUKLEINSÄUREN AUS EXTREM GERINGEN MENGEN SOWIE SEHR STARK VERUNREINIGTEN UNTERSCHIEDLICHSTEN AUSGANGSMATERIALIEN**
UNIVERSAL PROCESS FOR ISOLATING AND PURIFYING NUCLEIC ACIDS FROM EXTREMELY SMALL AMOUNTS OF HIGHLY CONTAMINATED VARIOUS STARTING MATERIALS
PROCEDE UNIVERSEL D'ISOLEMENT ET DE PURIFICATION D'ACIDES NUCLEIQUES A PARTIR DE QUANTITES EXTREMEMENT REDUITES DE DIFFERENTS MATERIAUX DE DEPART FORTEMENT CONTAMINES

(30) Priorität: 14.06.1994 DE 4422040; 14.06.1994 DE 4422044; 30.12.1994 DE 4447015
(43) Veröffentlichungstag der Anmeldung: 02.04.1997
(73) Patentinhaber: Invitek GmbH, 13125 Berlin (DE)
(72) Erfinder: HILLEBRAND, Timo, D-12619 Berlin (DE); BENDZKO, Peter, D-12623 Berlin (DE); PETERS, Lars-Erik, D-10367 Berlin (DE)
(74) Vertreter: Baumbach, Friedrich
(86) Internationale Anmeldenummer: DE9500787
(87) Internationale Veröffentlichungsnummer: WO9534569

(56) Entgegenhaltungen:
- EP-A- 0 268 946
- EP-A- 0 389 063
- WO-A-89/01035
- WO-A-92/07863
- WO-A-93/11218
- WO-A-93/11221
- WO-A-95/01359
- STN International, Derwent Information Ltd, WPIDS accession no. 95-001705, Sumitomo Metal Ind Ltd: "Reagent for extracting DNA from biological samples - comprises protein dena- turing agent and aq.soln. of protein-dissolving agent..", JP 06289016 A 941018 (9501)

## Beschreibung

Die Erfindung betrifft ein Verfahren zur Isolierung und Reinigung von Nukleinsäuren aus extrem geringen und u.U. auch sehr stark mit organischen wie auch anorganischen Komponenten kontaminierten unterschiedlichen biologischen und anderen Ausgangsmaterialien. Es ist für eine Vielzahl von biologisch-, molekularbiologisch-, forensisch-, medizinisch- analytisch- sowie biochemisch arbeitenden Laboratorien von großer Bedeutung. Damit sind Anwendungsgebiete der Erfindung die forensische Medizin, medizinische Diagnostik, Molekularbiologie, Biochemie, Gentechnik und alle anderen angrenzenden Gebiete.
Üblicherweise werden Nukleinsäuren aus Zellen und Geweben dadurch gewonnen, daß die Ausgangsmaterialien unter stark denaturierenden und reduzierenden Bedingungen, teilweise auch unter Verwendung von proteinabbauenden Enzymen aufgeschlossen, die austretenden Nukleinsäurefraktionen über Phenol-/Chloroform-Extraktionsschritte gereinigt und die Nukleinsäuren mittels Dialyse oder Ethanolpräzipitation aus der wäßrigen Phase gewonnen werden (Sambrook, J., Fritsch, E.F. und Maniatis, T., 1989, CSH, "Molecular Cloning" ).
Diese "klassischen Verfahren" zur Isolierung von Nukleinsäuren aus Zellen und besonders aus Geweben sind sehr zeitaufwendig ( teilweise länger als 48 h ), erfordern einen erheblichen apparativen Aufwand und sind darüber hinaus auch nicht unter Feldbedingungen realisierbar. Außerdem sind solche Methoden auf Grund der verwendeten Chemikalien wie Phenol und Chloroform in einem nicht geringen Maße gesundheitsgefährdend.
Verschiedene alternative Verfahren zur Isolierung von Nukleinsäuren aus unterschiedlichen biologischen Ausgangsmaterialien ermöglichen, die aufwendige und gesundheitsschädigende Phenol-/Chloroform-Extraktion von Nukleinsäuren zu umgehen sowie eine Reduzierung der zeitlichen Aufwendungen zu erreichen.
Alle diese Verfahren basieren auf einer von Vogelstein und Gillespie (Proc. Natl. Acad. Sci. USA, 1979, 76, 615-619) entwickelten und erstmals beschriebenen Methode zur präparativen und analytischen Reinigung von DNS-Fragmenten aus Agarosegelen. Die Methode kombiniert die Auflösung der die zu isolierende DNS-Bande enthaltende Agarose in einer gesättigten NaJ- Lösung mit einer Bindung der DNS an Glaspartikel in Gegenwart dieses chaotropen Salzes. Die an die Glaspartikel fixierte DNS wird anschließend mit einer Waschlösung (20 mM Tris HCl [pH 7,2]; 200mM NaCl; 2 mM EDTA; 50% v/v Ethanol) gewaschen und abschließend von den Trägerpartikeln abgelöst.
Diese Methode erfuhr bis heute eine Reihe von Modifikationen und wird zum gegenwärtigen Zeitpunkt für unterschiedliche Verfahren der Extraktion und Reinigung von Nukleinsäuren aus unterschiedlichen Herkünften angewendet (Marko, M.A., Chipperfield, R. und Birnboim, H.G., 1982, Anal. Biochem., 121, 382-387).
Darüberhinaus existieren heute weltweit auch eine Vielzahl von Reagenziensystemen, vor allem zur Reinigung von DNS-Fragmenten aus Agarosegelen und für die Isolierung von Plasmid DNS aus bakteriellen Lysaten, aber auch für die Isolierung von längerkettigen Nukleinsäuren (genomische DNS, zelluläre Gesamt-RNS) aus Blut, Geweben oder auch Zellkulturen .
So ist aus EP-A-389 063 ein Verfahren zur Isolierung von Nukleinsäuren bekannt, nach dem Trägermaterialien mit einer Partikelgröße zwischen 50 und 500 000 nm eingesetzt werden. Alle diese kommerziell verfügbaren Kits basieren auf dem hinlänglich bekannten Prinzip der Bindung von Nukleinsäuren an mineralische Träger unter Anwesenheit hochmolarer Lösungen unterschiedlicher chaotroper Salze und verwenden als Trägermaterialien Suspensionen feingemahlener Glaspulver (z.B. Glasmilk , BIO 101, La Jolla, CA), Diatomenerden (Fa.Sigma) oder auch Silicagele. (Diagen, DE 41 39 664 Al).
Jedoch verfügen weder klassische Glasmilch- oder Diatomenerden-Suspensionen als auch auf chromatographischen Säulen fixierte Silicagele über für die Isolierung von geringsten Mengen an Nukleinsäuren notwendigen physikalischen Vorraussetzungen.
Weiterhin wirken sich solche physikalische Charakteristika, wie poröse oder strukturierte wie auch relativ geringe aktive Oberflächen dieser Trägermaterialien außerdem ungünstig auf die Entfernung von Kontaminanten aus.
So ist es z.B.nicht möglich, PCR-taugliche DNS aus Speichelproben unter Verwendung von Glasmilch zu isolieren (Ochert, A.S. et.al.; 1993, PCR Methods and Application, 3, 6, 365-368). Die an die Glasprtikel gebundene DNS kann nicht ausreichend gewaschen werden bzw. bindet wahrscheinlich auch selbst z.B. niedermolekulare Zuckerverbindungen, so das diese im Speichel enthaltenen Kontaminanten auch in der finalen DNS zu finden sind und nachfolgende enzymatische Reaktionen (z.B. PCR) inhibieren.
Nach dem Stand der Technik existiert auch keine effiziente und schnelle Methode, welche es gestattet genomische (oder auch bakterielle oder virale) DNS aus Stuhlproben zu isolieren. Stuhlmaterial als biologisches Ausgangsmaterial ist extrem stark verunreinigt und erfordert sehr hohe Anforderungen an ein DNS-Isolierungssystem.
Zum gegenwärtige Zeitpunkt angewendete Methoden zur Isolierung von DNS aus Stuhlproben benötigen teilweise einige Tage und schließen aufwendige Proteinase K-Verdauungen und Phenol/Chloroform Extraktionen sowie Ethanolpräzipitationen ein und benötigen zusätzlich nochmals eine Reinigung der schon isolierten Nukleinsäuren unter Verwendung der bekannten DNS-Bindung an Glaspartikel.
Ein weiteres Problem der z.Z. für die Isolierung genomischer (und damit langkettiger) DNS verwendeten Glasmaterialien oder poröse Silicagele enthaltende Säulen liegt in der sehr starken mechanischen Beanspruchung ( Scherung ) der hochmolekularen DNS.
Sowohl mit Glasmilch oder- Diatomen-Suspensionen, als auch mit Silicagel enthaltenen Minisäulen, isolierte genomische DNS zeigt gelelektrophoretisch oftmals (vor allem bei der Verwendung von Minisäulensystemen) deutlich sichtbare Degradierungen. Die Ursache liegt dabei zum einen in der mechanischen Beanspruchung der DNA bei der Passage durch die Poren der Silicagele, bzw. durch die keine homogene Teilchenkonsistenz aufweisenden Glaspulver oder Diatomenerden enthaltenen Trägersuspensionen (Diatomenerde z.B. enthält z.B. auch sehr scharfkantige Partikel und ist zudem auch nicht größenhomogen). Somit führt jeder Waschschritt oder Zentrifugationsschritt zu einer Degradierung der DNS.
Auch ist die Bindung von zellulärer Gesamt-RNS an z.B. silicageltragende Säulen bekannt und als Reagenzienssystem auch verfügbar , realisiert dabei aber nicht eine vollständige Isolierung von zellulärer Gesamt-RNS, da kleinere RNA Spezies (<200 bp) nicht mehr isoliert werden können. Somit kann z.B. eine solche RNS nicht mehr erfolgreich für DDRT-PCR Anwendungen (Isolierung sämtlicher mRNA Spezies einer Zelle) eingesetzt werden, da von vornherein kleine RNS-Spezies nicht vorhanden sind. Desweiteren besteht mit solchen Systemen auch nicht die Möglichkeit der Isolierung von RNS aus extrem geringen Mengen an Ausgangsmaterialien. Obwohl Verfahren der Isolierung, wie auch Reinigung von Nukleinsäuren über das bekannte Prinzip der Bindung der Nukleinsäuren an mineralische Materialien, mittlerweile weitverbreitet sind, werden eine Reihe von speziellen Anwendungen der Isolierung von Nukleinsäuren, mit bisher bekannten Isolierungssystemen (und den verwendeten Trägermaterialien) nicht oder in keiner Weise zufriedenstellend gelöst.
Dies betrifft:
1. die Isolierung von Nukleinsäuren (genomische DNS, total RNS) aus extrem geringen Mengen an Ausgangsmaterialien (z.B. <0.5mg Gewebematerial; <0.5µl Blut oder Blutspuren auf Kleidungsstücken; <5µl Speichel; <10³ Zellen),
2. die Verfügbarkeit eines universellen Systems für die Isolierung von Nukleinsäuren aus einem sehr breiten Spektrum unterschiedlichster Ausgangsmaterialien (d.h. Isolierung von Nukleinsäuren aus 'einfachen Ausgangsmaterialien' wie Zellkulturen oder Vollblut wie auch aus extrem schwierigen Ausgangsmaterialien wie Uraltknochen oder Stuhlmaterialien),
3. die Isolierung von Nukleinsäuren aus stark kontaminiertem Ausgangsmaterial in einer Qualität, die es erlaubt, die isolierten Nukleinsäuren auch als Substrat für enzymatische Nachfolgereaktionen (z.B. PCR) auch mit Erfolg einsetzen zu können.

Gerade solche kontaminationsbeladenen Ausgangsmaterialien sind für bestimmte klinisch relevante Fragestellungen für die Diagnostik, für forensische Untersuchungen bzw. für die Klärung evolutionsbiologischer Fragestellungen von großer Bedeutung. Es handelt sich dabei vor allem um solche `interessierenden' Ausgangsmaterialien wie Knochen oder Blutspuren auf Kleidungsstoffen (forensische Medizin), Uraltknochen (Evolutionsbiologie), Speichel , Bronchialauswurfmaterial und Stuhlproben (medizinische Diagnostik). Wie schon aufgeführt ist es z.B. nicht möglich amplifikationsfähige DNS aus Speichelproben unter Verwendung von Glasmilch zu isolieren. Noch komplizierter stellt sich dieses Problem dar, wenn DNS aus Stuhlproben isoliert werden soll. Nach dem gegenwärtigen Stand der Technik muß eindeutig festgestellt werden, daß es noch kein funktionierendes schnelles Isolierungsverfahren gibt (weder kommerziell verfügbar, noch publiziert), um amplifikationsfähige Nukleinsäuren zu isolieren. Bisher sind für eine Isolierung von DNS aus einem solchen Ausgangsmaterial extrem arbeits-und-zeitaufwendige multiple Reinigungsschritte notwendig. Solche aufwendigen Prozeduren sind notwendig, da die Vielzahl der in Stuhlproben enthaltenen Kontaminationen mit allen bisher bekannten Methoden nicht anders entfernt werden konnten. Eine direkte und damit sehr schnelle Isolierung amplifikationsfähiger Nukleinsäure aus Stuhlproben über die Bindung der Nukleinsäuren an Trägermaterialien ist bisher nicht bekannt und bedeutet damit einen sehr großen Nachteil, da Stuhlmaterial als Quelle für zu isolierende Nukleinsäuren (vor allem genomische DNS aus abgeschilferten epithelialen Zellen der Darmwand) für die routinemäßige Gendiagnostik nicht verfügbar ist.

Die Aufgabe der Erfindung besteht darin, ein universelles Verfahren zur Isolierung und Reinigung von Nukleinsäuren bereitzustellen, das diese speziellen Anwendungen gestattet.

Überraschenderweise wurde gefunden, daß mit dem erfindungsgemäß eingesetzten Trägermaterial und unter der Verwendung unterschiedlicher chaotroper Salze alle diese Anforderungen in hervorragender Weise erfüllt werden.
Das erfindungsgemäße Verfahren wird gemäß den Ansprüchen 1-14 realisiert. Es ist dadurch gekennzeichnet, daß die Nukleinsäuren enthaltenden Ausgangsmaterialien lysiert, das Lysat mit einem nichtporösen und unstrukturierten, hochdispersen sowie homogenen chemisch reinen SiO₂- Träger inkubiert, der Träger mit den gebundenen Nukleinsäuren abgetrennt und mit Pufferlösung gewaschen und nachfolgend die Nukleinsäuren mit einem Puffer geringer Salzkonzentration vom Träger abgelöst werden.
Die Lyse von Nukleinsäuren enthaltenden Materialien erfolgt mit Puffern, die chaotrope Salze hoher Ionenstärke enthalten.
Die Fixierung der Nukleinsäuren erfolgt an der Oberfläche der SiO₂-Partikel, welche eine Korngröße von 7-40 nm bei einer aktiven Oberfläche von 50-300 m²/g, vorzugsweise einen Partikeldurchmesser von 40 nm, bei einer aktiven Oberfläche von ca. 50 m²/g aufweisen, unter Anwesenheit chaotroper Salze hoher Ionenstärken.
Als chaotrope Salze werden vorzugsweise Guanidinhydrochlorid, Guanidinthiocyanat, Lithiumchlorid, Natriumjodid, Kaliumjodid, Natriumperchlorat oder Lithiumchlorid/ Harnstoff-Gemische mit Ionenstärken > 4M eingesetzt.

Damit wird es möglich, Nukleinsäuren aus
a) extrem geringen Mengen an Nukleinsäuren enthaltenden Ausgangsmaterialien
b) aus sehr 'schwierigen' und stark mit organischen und anorganischen Verunreinigungen kontaminierten unterschiedlichen biologischen und anderen Ausgangsmaterialien wie z.B. Stuhlproben, Knochen u.a.
in einer Qualität und Quantität zu isolieren, welche nachfolgende enzymatische Manipulationen mit den isolierten Nukleinsäuren möglich werden läßt.

Es zeigte sich ebenfalls überraschenderweise, daß über die Auswahl des verwendeten chaotropen Puffers in Kombination mit dem verwendeten Trägermaterial eine Selektivität der Bindung von entweder DNS oder RNS realisiert wird. Damit kann allein durch die Wahl des für die Lyse des Ausgangsmaterials verwendeten chaotropen Salzes, das Trägermaterial für die Isolierung von DNS oder für die Isolierung RNS eingesetzt werden, wobei im methodischen Verfahrensablauf keinerlei Veränderungen durchzuführen sind. Ein solches Verhalten eines für die Bindung von Nukleinsäuren eingesetzten Materials ist bisher noch nicht beschrieben worden.

Das Verfahren zur Isolierung von Nukleinsäuren ist sehr einfach handhabbar, benötigt nur geringe apparative Ausrüstungen, bedarf keiner enzymatischen Vorbehandlung des Probenmaterials (z.B. Proteinase K Verdauung), verzichtet auf den Einsatz einer toxischen Phenol-/Chloroform-Extraktion, benötigt keine Ethanolpräzipitation und läßt sich somit mit geringem zeitlichen Aufwand realisieren, was es gestattet, einen großen Probenumfang zu bearbeiten.

Das erfindungsgemäß eingesetzte Trägermaterial mit seinen physikalischen Eigenschaften ist ideal für die Isolierung und Reinigung von Nukleinsäuren aus extrem geringen Mengen an unterschiedlichsten Ausgangsmaterialien, wie auch aus sehr stark mit Verunreinigungen kontaminierten Ausgangsmaterialien. Die der Erfindung zugrunde liegenden Eigenschaften des ausgewählten Trägermaterials, welche im folgenden im Vergleich zu anderen Trägermaterialien dargestellt werden, ermöglicht damit erstmalig die Entwicklung eines universellen Isolationssystems für Nukleinsäuren (universell im Sinne der Isolierung von sowohl DNS als auch RNS, der Isolierung der Nukleinsäuren aus allen Nukleinsäuren enthaltenden biologischen und anderen Ausgangsmaterialien, der Isolierung von Nukleinsäuren aus extrem geringen Mengen an Ausgangsmaterialien, sowie der Isolierung von Nukleinsäuren aus sehr stark mit Verunreinigungen kontaminierten Ausgangsmaterialien). Wie weiterhin aufgezeigt wird, ist ein solches universelles System nicht mit herkömmlich verwendeten Glasmaterialien (Glasmilch,Glaspulver o.ä.) realisierbar.

Das in der Erfindung verwendete Trägermaterial unterscheidet sich hinsichtlich seiner physikalischen Charakteristika grundlegend von anderen für die Isolierung von Nukleinsäuren verwendeten Trägermaterialien, bei welchen es sich in der Regel nicht um chemisch reines SiO₂, sondern um poröse oder nichtporöse Glasmaterialien z.B. auf der Basis von Borsilikatglas (Glasmilch) oder aus pflanzlichen mineralischen Zellwandkomponenten (Diatomenerden), welche als chromatographische Materialien speziell für Anwendungen in der Nukleinsäurenreinigung kommerziell verfügbar sind, und damit die Basis für die Herstellung von (auch der kommerziell verfügbaren) Suspensionen bilden. Alle diese Trägermaterialien sind sehr gut geeignet für Standardanwendungen, zeigen aber auf Grund ihrer physikalischen Struktur erhebliche Nachteile bzw. Grenzen bei der Isolierung von Nukleinsäuren aus den oben beschriebenen Ausgangsmaterialien.

Das im erfindungsgemäßen Verfahren verwendete Trägermaterial ist wesentlich kleiner als bisher verwendete Materialien. Damit einhergehend ist die für die Fixierung der Nukleinsäuren verfügbare aktive Trägeroberfläche sehr viel größer.
Gerade die nur im nm-Bereich definierte Größe der Trägerpartikel als auch die daraus resultierende sehr hohe spezifische Oberfläche ermöglichen die Isolierung von Nukleinsäuren aus extrem geringen Mengen an Ausgangsmaterialien..
Dabei entspricht das dem erfindungsgemäßen Verfahren zugrunde liegende Ziel, die Isolierung von Nukleinsäuren aus extrem geringen Mengen an unterschiedlichen Ausgangsmaterialien, letzlich auch einer neuen Strategie, die es ermöglichen soll, eine Verbindung von schon existierenden als sich noch in der Entwicklung befindlichen Methoden der sog. nichtinvasiven medizinischen Probengewinnung (Mikromengen an Mikrobiopsiematerialien; Gewinnung von epithelialen Lungenzellen mit speziell konstruierten 'Ausatemluftsammlern' u.a.) und Methoden der medizinischen Diagnostik herzustellen. Bisher war es nicht möglich, aus extrem geringen Nukleinsäuren enthaltenen Proben (z.B. Biopsiematerialien, Lungenflüssigkeit) Nukleinsäuren für eine weitere Diagnostik zu isolieren.
Die mit dem erfindungsgemäßen Verfahren realisierte Isolierung von Nukleinsäuren aus solchen extrem geringen Mengen an Ausgangsmaterialien eröffnet damit den neuen nichtinvasiven Methoden in der medizinischen Probengewinnung erstmalig neue potentielle Anwendungsgebiete.

Ein weiterer bedeutender Vorteil der im erfindungsgemäßen Verfahren verwendeten Trägerpartikel besteht in der direkten Bindung und damit der direkten Exponierung der Nukleinsäuren auf der Trägeroberfläche. Im Gegensatz dazu befinden sich die zu isolierenden Nukleinsäuren bei verwendeten chromatographischen Systemen (z.B. DE 41 39 664 A1), welche eine Kombination von porösen Anionenaustauschern und mineralischen Trägermaterialien verwenden, nicht auf der Oberfläche, sondern innerhalb der Poren.

Eine solche Lokalisation reduziert sehr stark die Möglichkeit intensiven Waschens, und damit des Entfernens unterschiedlicher Kontaminanten. Ebenso ermöglichen auch Glasmilch-Suspensionen, Suspensionen aus Diatomenerde oder in der Patentschrift DE 41 39 664 Al beschriebene poröse oder nichtporöse Matrices ( im Größenbereich von 1µm-250µm, vorzugsweise 10-30µm) auf Grund der viel größeren Partikelgröße (wie auch auf Grund dessen, daß es sich teilweise bei den Trägerpartikeln nicht um homogene Gemische handelt) und damit verbunden geringeren Trägeroberfläche kein effektives Waschen der gebundenen Nukleinsäuren. Gerade das Fehlen eines solchen intensiven Waschens wird dann zum Problem, wenn Nukleinsäuren aus Ausgangsmaterialien welche enorme Mengen ganz unterschiedlicher Kontaminationen, wie z.B Schleimstoffe, Farbstoffe, niedermolekulare Zuckerverbindungen u.a. , beinhalten, isoliert werden sollen.

Das erfindungsgemäße Verfahren mit dem verwendeten Trägermaterial erlaubt erstmals die Möglichkeit einer direkten DNS-Isolierung aus abgeschilferten Darmwandzellen ohne aufwendige Proteinase K-Verdauungen, Phenol/Chloroform Extraktionen sowie Ethanolpräzipitationen. Nach Abtrennung unlöslicher Komponenten aus der Stuhlprobe und Zugabe ein chaotropes Salz enthaltenen Puffers wird die DNS direkt an das Trägermaterial gebunden, gewaschen und vom Träger eluiert. Diese Methode ist extrem schnell, (ca. 1 h) und liefert genomische DNS in exzellenter Qualität, die problemlos für PCR-Applikationen verwendet werden kann. Eine solche schnelle Methode der Isolierung amplifikationsfähiger DNS aus Stuhlmaterial ist bisher mit keinem anderen DNS-Isolierungssystem erreicht worden. Dies ermöglicht z.B. erstmalig die Durchführung eines schnellen und reproduzierbaren Nachweises von Kolonkarzinom assoziierten Punktmutationen im Proto-Onkogen Kras als ein zukünftig bedeutsames Gen-Diagnostikverfahren und wird damit bedeutsam für protektive Maßnahmen bei Risikogruppen bzw. in der Früherkennung von Kolonkarzinomen bzw. auch Pankreaskarzinomen.
Eine solche routinemäßig durchführbare Gen-Diagnostik war bisher auf Grund des Fehlens einer geeigneten Methode der Isolierung und Reinigung von genomischer DNS unmöglich.

Das im erfindungsgemäßen Verfahren verwendete und unter solchen Gesichtspunkten ausgwählte Trägermaterial reduziert auch sehr stark (im Gegensatz zu anderen Trägermaterialien bzw. Säulensystemen) mechanische Beanspruchungen der DNS auf Grund der Feinheit der Partikel und der Homogenität, bzw. auch dadurch, daß es sich eben nicht um zerstanzte Glasmaterialien bzw. Diatomenerden handelt. Somit, und dieses zeigt sich ebenfalls sehr eindeutig in einer gelelektrophoretischen Darstellung, weist die isolierte DNS einen sehr hohen Grad an Integrität auf und ist damit von vergleichbarer Qualität wie mit der sehr schonenden klassischen Phenol/Chloroform Extraktionsmethode gewonnene DNS. Genomische DNS hoher Integrität ist vor allem dann gefordert, wenn die DNS als Substrat für LA/XL-PCR Anwendungen oder DNS-Fingerprint Techniken einqesetzt werden soll.

Ein weiterer Vorteil des erfindungsgemäßen Verfahrens besteht darin, daß das verwendete Trägermaterial auch für die Isolierung von Ribonukleinsäuren (RNS) in idealer Weise geeignet ist und ermöglicht im Gegensatz zu z.Z. für die Isolierung von RNS Verwendung findenden mit Silicagel-Matrices beladenen Minisäulensystemen (Fa. Diagen) auch eine Isolierung von vollständiger zellulärer Gesamt-RNS, d.h. auch die Isolierung sehr kleiner RNS-Spezies.
Das erfindungsgemäße Verfahren gestattet sowohl die Isolierung von zellulärer Gesamt-RNS einschließlich der tRNS und 5S RNS Fraktionen (und damit auch aller kleinen RNS-Spezies), als auch die Isolierung von RNS aus wenigen hundert Zellen und damit aus extrem geringen Ausgangsmengen. Es ist damit in ausgezeichneter Weise für Expressionsuntersuchungen von sehr geringen Zellmengen geeignet, zumal die gesamte Prozedur der Isolierung der Gesamt-RNS aus solchen Ausgangsmaterialien nur ca. 20 Minuten in Anspruch nimmt. Ferner sorgt wiederum die sehr große spezifische Oberfläche der Trägerpartikel für eine sehr hohe Bindungseffizienz, so das theoretisch 100% der in den verschiedenen Ausgangsmaterialien vorhandenen RNS auch isoliert werden können. Dies ist vor allem dann bedeutsam, wenn quantitative PCR-Anwendungen z.B. in der Hepatitis C Diagnostik entwickelt werden sollen, um Aussagen über den Verlauf des Virustiters im Blut zu erhalten. Der Einsatz einer quantitativen PCR für die Beantwortung solcher Fragestellungen hängt damit in sehr starkem Maße vom verwendeten RNS Isolierungssystem ab.
Die Bindung von RNS-Molekülen an das verwendete Trägermaterial wird durch die Verwendung von hochmolarem Lithiumchlorid, vorzugsweise 10-molarem, enthaltenem Lyse/Bindungspuffer realisiert, welcher mit einer viel höherer Affinität die Bindung von RNS-Molekülen gegenüber von DNS-Molekülen an das verwendete Trägermaterial vermittelt und wobei die Lyse und Bindung der Ribonukleinsäuren an den SiO₂-Träger im selben Reaktionsgefäß erfolgt.
Wie schon aufgeführt wird alleine durch die Wahl des Lyse/Bindungspuffers eine unterschiedliche Bindungsspezifität erreicht, so daß das selbe Trägermaterial eben auch für die Isolierung von RNS-Molekülen eingesetzt werden kann. Es verhält sich damit auch gänzlich different gegenüber existierenden theoretischen Vorstellungen physiko-chemischen Mechanismen der Bindung von Nukleinsäuren an mineralische Materialien unter chaotropen Bedingungen, wonach nach einer durch die chaotropen Salze hervorgerufene Zerstörung der Hydrathüllen von Nukleinsäuren eine Adsorbtion dieser an mineralische Matrices erfolgt. Dies bedeutet nach der theoretischen Darstellung aber auch, das einzelsträngige DNS bzw. RNS so stark an die mineralischen Materialien gebunden werden, daß eine Elution der RNS nur bei sehr hohen Temperaturen und damit auch unter Schädigung der RNS erfolgen kann. Bei Verwendung des im erfindungsgemäßen beschriebenen Trägermaterials und der Verwendung von Litiumchlorid sowohl zur Lyse als auch Realisierung der Bindung der RNS an das Trägermaterial, kann diese wieder problemlos mit DEPC (Diethylpy rocarbonat) behandeltem Wasser vom Träger eluiert werden.

Ein weiterer entscheidender Vorteil des erfindungsgemäßen Verfahrens, vor allem im Zusammenhang mit der Isolierung langkettiger genomischer DNS wie auch zellulärer Gesamt-RNS, besteht darin, daß die Methode keinerlei enzymatische Vorbehandlungen des Ausgangsmaterials (Proteinasebehandlungen) benötigt. Der je nach Isolierungsart eingesetzte Lysepuffer vermittelt neben seiner proteindenaturierenden Wirkung (und in diesem Zusammenhang auch Inaktivierung endogener und exogener DNasen oder RNasen) auch die Bindung der Nukleinsäuren an das Trägermaterial. Dies ermöglicht z.B. die Isolierung genomischer DNS aus einer Monolayer Zellkultur, einer Gewebeprobe oder aus Vollblut (z.B. mit 1x10⁵ Zellen, 0.5 mg Gewebe, 100µl Vollblut) in weniger als 30 Minuten. Sowohl Lyse als auch Bindung der Nukleinsäuren erfolgen unter denselben Pufferbedingungen und im selben Reaktionsgefäß. Dies bedeutet damit auch einen entscheidenden zeitlichen Vorteil gegenüber anderen Isolierungssystemen langkettiger Nukleinsäuren aus solchen biologischen Ausgangsmaterialien.

Neben der Isolierung der beschriebenen längerkettigen DNS (genomisch DNS) bzw. RNS eignet sich der eingesetzte Träger auch für die Isolierung und Reinigung von Nukleinsäuren aus TAE- bzw. TBE- Gelen, PCR-Fragmenten direkt aus den Reaktionsgemischen (einschließlich vorhandenen Mineralöls), als auch für die Isolierung von Plasmid DNA aus bakteriellen Lysaten. Wobei auf diese Möglichkeiten nicht weiter eingegangen werden soll. Auch hierbei realisiert das eingesetzte Trägermaterial sehr hohe Rückgewinnungsraten von DNS-Fragmenten aus Gelen oder PCR-Ansätzen in Größenbereichen von 60 bp - 50 kbp bzw. sehr hohe Ausbeuten qualitativ hochwertiger Plasmid DNS.

Die Größe der zu isolierenden oder zu reinigenden Nukleinsäuren umfaßt bevorzugt den Bereich von 50 Nukleotiden bis zu 60 000 Nukleotiden.

Bei allen beschriebenen Anwendungen vermittelt der jeweils verwendete Lysepuffer (je nach Anwendung bestehend aus Guanidinthiocyanat, NaI, Guanidinhydrochlorid oder Litiumchlorid und entsprechenden Detergenz-Zusätzen) auch jeweils die Bindung der Nukleinsäuren an das Trägermaterial.
Alle mit den erfindungsgemäßen Verfahren isolierten und am Träger fixierten Nukleinsäuren aus unterschiedlichen Ausgangsmaterialien werden mit einem Waschpuffer ( 50mM NaCl; 10mM Tris HCl; lmM EDTA; 70% v/v ) mehrmals gewaschen. Der verwendete Waschpuffer unterscheidet sich von dem in der von Vogelstein und Gillespie beschriebenen Ausgangsarbeit durch eine geringere Salz- und höhere Ethanolkonzentration. Eine solche Waschpufferzusammensetzung erlaubt ein intensiveres Waschen ohne Verlust an gebundenen Nukleinsäuren.
Die Elution der Nukleinsäuren erfolgt vorzugsweise in einem Elutionspuffer niedriger Ionenstärke bestehend aus 10mM Tris HCl; 0. 1mM EDTA, anderen Niedrigsalzpuffern oder in DEPC behandeltem Wasser bei einer Temperatur von 48-56 °C, vorzugsweise 52°C, innerhalb von maximal 5 min.

Die isolierten Nukleinsäuren sind für eine Vielzahl weiterer molekularbiologischer bzw. biochemischer Methoden verfügbar, wie z.B. PCR/RT-PCR und spezielle PR-Applikationen (LAXL-PCR, RAPD PCR-Fingerprinting u.a.) Spaltung mit Restriktionsendonukleasen, Klonierungen, Sequenzierungen, in vitro- Transkription, radioaktiven Markierungen, Hybridisierungsverfahren u.a..

Die Erfindung wird nachfolgend an Ausführungsbeispielen näher erläutert, die die Erfindung jedoch nicht begrenzen sollen.

### 1. Isolierung genomischer DNS aus einer eukaryontischen monolayer Zellkultur, welche auf 96-Well- Mikrotiterplatten kultiviert wurde (ca. 5x10³ Zellen)

Entfernung des Zelkulturüberstandes und zweimaliges kurzes Spülen der Zellen mit 1xPBS.
Zugabe von 500µl Lysepuffer (Guanidinthiocyanat; N-Lauryl-Sarcosyl; DTT; Natriumcitrat) direkt auf das Well und Überführung der Zell-Lyse-Suspension in ein 1.5 ml Eppendorf-Zentrifugengefäß. Zugabe von 10µl der aus dem verwendeten Trägermaterial hergestellten Suspension zur Zell-Lyse-Suspension, kurzes Vortexen, Inkubation für 5 Minuten in einem Eisbad und anschließende Pelletierung des Trägermaterials durch kurze Zentrifugation in einer Tischzentrifuge (10 Sekunden). Die am Trägerpellet gebunden genomische DNS wird anschließend mit Waschpuffer (50 mM NaCl; 10 mM Tris HCl; 1 mM EDTA; 70% v/v Ethanol) versetzt und 2-3 mal gewaschen und anschließend die genomischen DNS durch Zugabe eines Elutionspuffers (10 mM Tris; 0.1 mM EDTA) bei 52°C vom Trägermaterial abgelöst, der Träger durch kurze Zentrifugation von der eluierten genomische DNS abgetrennt und diese in ein neues Reaktionsgefäß überführt.

### 2. Isolierung zellulärer total RNS aus einer Hybridomazellsuspension (ca. 200µl; ca. 10³ Zellen)

Überführung der Zellsuspension in ein 1.5 ml Eppendorf Zentrifugengefäß und Zugabe von 500µl Lysepuffer (10 M LiCl, 2% Triton X-100). Inkubation für 5 min bei Raumtemperatur.
Zugabe von 10µl der aus dem verwendeten Trägermaterial hergestellten Suspension zur Zell-Lyse-Suspension, kurzes Vortexen, Inkubation für 5 Minuten in einem Eisbad und anschließende Pelletierung des Trägermaterials durch kurze Zentrifugation in einer Tischzentrifuge (10 Sekunden). Die am Trägerpellet gebunden RNS wird anschließend mit Waschpuffer (50 mM NaCl; 10 mM Tris HCl; 1 mM EDTA; 70% v/v Ethanol) versetzt und 2-3 mal gewaschen und anschließend die zelluläre Gesamt- RNS durch Zugabe von DEPC behandeltem H₂O (doppeltdestilliert) bei 52°C vom Trägermaterial abgelöst, der Träger durch kurze Zentrifugation von der eluierten zellulärern Gesamt-RNS abgetrennt und diese in ein neues Reaktionsgefäß überführt.

### 3. Isolierung genomischer DNS aus einer ca. 0.5µl Blutspur auf einem Papiertaschentuch

Zerschneiden des die Blutspur tragenden Bereiches des Papiertaschentuches und Überführen der Schnipsel in ein 1.5 ml Eppendorf Zentrifugengefäß.
Zugabe von 500µl Lysepuffer (Guanidinthiocyanat; N-Lauryl-Sarcosyl; DTT; Natriumcitrat) und Inkubation für einige Stunden bei Raumtemperatur.
Kurze Zentrifugation zur Abtrennung der unlöslichen Bestandteile, Überführung des Überstandes in ein neues Zentrifugengefäß und Zugabe von 10µl der aus dem verwendeten Trägermaterial hergestellten Suspension zur, kurzes Vortexen, Inkubation für 5 Minuten in einem Eisbad und anschließende Pelletierung des Trägermaterials durch kurze Zentrifugation in einer Tischzentrifuge (10 Sekunden). Die am Trägerpellet gebunden genomische DNS wird anschließend wiederum mit Waschpuffer (50 mM NaCl; 10 mM Tris HCl; 1 mM EDTA; 70% v/v Ethanol) versetzt und 2-3 mal gewaschen und anschließend die genomischen DNS durch Zugabe eines Elutionspuffers (10 mM Tris; 0.1 mM EDTA) bei 52°C vom Trägermaterial abgelöst, der Träger durch kurze Zentrifugation von der eluierten genomische DNS abgetrennt und diese in ein neues Reaktionsgefäß überführt.

### 4. Isolierung genomischer DNS aus Knochenmaterial

Überführung von ca. 100-250 mg von feinzermahlenem Knochenpulver in ein 2.0 ml Eppendorf Zentrifugengefäß. Zugabe von 1ml Lysepuffer (Guanidinthiocyanat; N-Lauryl-Sarcosyl; DTT; Natriumcitrat; 0.5 M EDTA) und Inkubation bei 56°C und leichtem Schütteln für 15-20h.
Zentrifugation bei 12-14 000 rpm und Überführung des Überstandes in ein neues Zentrifugengefäß.
Zugabe von 15µl der aus dem verwendeten Trägermaterial hergestellten Suspension, kurzes Vortexen, Inkubation für 5 Minuten in einem Eisbad und anschließende Pelletierung des Trägermaterials durch kurze Zentrifugation in einer Tischzentrifuge (10 Sekunden). Die am Trägerpellet gebunden genomische DNS wird anschließend wiederum mit Waschpuffer (50 mM NaCl; 10 mM Tris HCl; 1 mM EDTA; 70% v/v Ethanol) versetzt und 3 mal gewaschen und anschließend die genomischen DNS durch Zugabe eines Elutionspuffers (10 mM Tris; 0.1 mM EDTA) bei 52°C vom Trägermaterial abgelöst, der Träger durch kurze Zentrifugation von der eluierten genomische DNS abgetrennt und diese in ein neues Reaktionsgefäß überführt.

### 5. Isolierung von genomischer DNS aus Stuhlproben

Überführung von ca. 100 mg der Stuhlprobe in ein 2.0 ml Eppendorf Zentrifugengefäß und Zugabe einer 300µl einer Waschlösung (NaCl, EDTA, Tris HCl). Vortexen für 30 Sekunden und anschließende Zentrifugation bei 10 000 rpm für 2 min. Überführen des Überstandes in ein neues 1.5 ml Eppendorf Zentrifugengefäß und Zugabe von lml Lysepuffer (Guanidinthiocyanat; N-Lauryl-Sarcosyl; DTT; Natriumcitrat). Inkubation bei Raumtemperatur für 20-30 min.
Zugabe von 15µl der aus dem verwendeten Trägermaterial hergestellten Suspension, kurzes Vortexen, Inkubation für 5 Minuten in einem Eisbad und anschließende Pelletierung des Trägermaterials durch kurze Zentrifugation in einer Tischzentrifuge (10 Sekunden). Die am Trägerpellet gebunden genomische DNS wird anschließend wiederum mit Waschpuffer (50 mM NaCl; 10 mM Tris HCl; 1 mM EDTA; 70% v/v Ethanol) versetzt und 3mal gewaschen und anschließend die genomischen DNS durch Zugabe eines Elutionspuffers (10 mM Tris; 0.1 mM EDTA) bei 52°C vom Trägermaterial abgelöst, der Träger durch kurze Zentrifugation von der eluierten genomische DNS abgetrennt und diese in ein neues Reaktionsgefäß überführt.

### 6. Isolierung von genomischer DNS aus einer einzelnen Haarwurzel

Inkubation einer einzelnen Haarwurzel in einem Volumen von 500µl Lysepuffer (Guanidinthiocyanat; N-Lauryl-Sarcosyl; DTT; Natriumcitrat) für 30-60 min bei Raumtemperatur. Zugabe von 15µl der aus dem verwendeten Trägermaterial hergestellten Suspension, kurzes Vortexen, Inkubation für 5 Minuten in einem Eisbad und anschließende Pelletierung des Trägermaterials durch kurze Zentrifugation in einer Tischzentrifuge (10 Sekunden). Die am Trägerpellet gebunden genomische DNS wird anschließend wiederum mit Waschpuffer (50 mM NaCl; 10 mM Tris HCl; 1 mM EDTA; 70% v/v Ethanol) versetzt und 2-3 mal gewaschen und anschließend die genomischen DNS durch Zugabe eines Elutionspuffers (10 mM Tris; 0.1 mM EDTA) bei 52°C vom Trägermaterial abgelöst, der Träger durch kurze Zentrifugation von der eluierten genomische DNS abgetrennt und diese in ein neues Reaktionsgefäß überführt.

### 7. Aufreinigung von PCR-Fragmenten direkt aus dem PCR-Reaktionsgemisch

Zugabe von 150 µl einer Immobilisierungslösung (6 M NaI mit enthaltener Trägersuspension) direkt zum PCR-Reaktionsgemisch, einschließlich des überschichteten Mineralöls. Kurzes Vortexen und Inkubation für 3 min in einem Eisbad. Pelletierung des Trägermaterials durch kurze Zentrifugation in einer Tischzentrifuge (10 Sekunden). Das am Trägerpellet gebunden PCR-Produkt wird anschließend wiederum mit Waschpuffer (50 mM NaCl; 10 mM Tris HCl; 1 mM EDTA; 70% v/v Ethanol) versetzt und 2 mal gewaschen und anschließend die durch Zugabe eines Elutionspuffers (10 mM Tris; 0.1 mM EDTA) bei 52°C vom Trägermaterial abgelöst, der Träger durch kurze Zentrifugation vom eluierten PCR-Fragment abgetrennt und dieses in ein neues Reaktionsgefäß überführt.

## Patentansprüche

1. Verfahren zur Isolierung und Reinigung von Nukleinsäuren aus extrem geringen Mengen sowie sehr stark verunreinigten unterschiedlichsten Ausgangsmaterialien durch
a) Lyse von Nukleinsäuren enthaltenden Materialien mit Puffern, die chaotrope Salze hoher Ionenstärke enthalten,
b) Inkubation mit einem hochdispersen, nichtporösen und nichtstrukturierten sowie homogenen SiO₂-Träger, wobei die SiO₂-Partikel eine Korngröße von 7 - 40 nm aufweisen bei einer aktiven Oberfläche von 50 - 300 m²/g,
c) Abtrennung der trägerfixierten Nukleinsäuren vom Lysat,
d) Waschung der auf der Oberfläche des Trägers fixierten Nukleinsäuren mit einem Waschpuffer und
e) Ablösung der Nukleinsäure vom Träger mit einem Puffer geringer Salzkonzentration.

2. Verfahren nach Anspruch 1, dadurch gekennzeichnet, daß als chaotrope Salze Guanidinhydrochlorid, Guanidinthiocyanat, Lithiumchlorid, Natriumjodid, Kaliumjodid, Natriumperchlorat oder Lithiumchlorid/ Harnstoff-Gemische mit Ionenstärken > 4M eingesetzt werden.

3. Verfahren nach Anspruch 1 und 2, dadurch gekennzeichnet, daß als Trägermaterial hochdisperses, nichtporöses und nichtstrukturiertes sowie homogenes chemisch reines SiO₂ mit einer Korngröße von 40 nm bei einer spezifischen Oberfläche von 50 m²/g eingesetzt wird.

4. Verfahren nach Anspruch 1 bis 3, dadurch gekennzeichnet, daß die Lyse des die Nukleinsäuren enthaltenden Ausgangsmaterials und Bindung der Nukleinsäuren an die Trägerpartikel als 'single step' Verfahren im selben Reaktionsgefäß erfolgt.

5. Verfahren nach Anspruch 1 bis 4, dadurch gekennzeichnet, daß der Träger mit den gebundenen Nukleinsäuren vom übrigen Lysat durch einen kurzen Zentrifugationsschritt abgetrennt wird.

6. Verfahren nach Anspruch 1 bis 5, dadurch gekennzeichnet, daß die trägerfixierten Nukleinsäuren mit einem Waschpuffer, bestehend aus 50 mM NaCl, 10 mM Tris-HCl und 1 mM EDTA sowie 70% Ethanol, gewaschen werden.

7. Verfahren nach Anspruch 1 bis 6, dadurch gekennzeichnet, daß die trägerfixierten Nukleinsäuren mit einem Puffer niedriger Ionenstärke, bestehend aus 10 mM Tris-HCl, 0.1 mM EDTA, anderen Niedrigsalzpuffern oder DEPC behandelten bei einer Temperatur von 48 - 56°C, vorzugsweise 52°C, eluiert werden.

8. Verfahren nach Anspruch 1 bis 7, dadurch gekennzeichnet, daß es als batch-Verfahren durchgeführt wird.

9. Verfahren nach Anspruch 1 bis 8, dadurch gekennzeichnet, daß die Nukleinsäure aus Bakterien, Zellkulturen, intakten oder gefrorenen Gewebeproben, Gewebe-schnitten, Spermien, Körperflüssigkeiten, Stuhlproben, Viren, Pflanzenzellen, Hefezellen, musealen biologischen Trockenpräparaten, aus unterschiedlichen forensischen Ausgangsmaterialien, wie Blutspuren auf Kleidungsstücken, an Baumrinde u.a., aus Haaren, aus Speichel, aus Knochen oder anderen biologischen Quellen oder aus Amplifikationsreaktionen, wie PCR oder ähnlichen Reaktionen, stammt oder daß es sich um markierte Nukleinsäuren handelt.

10. Verfahren nach Anspruch 1 bis 9, dadurch gekennzeichnet, daß durch das spezielle für die Bindung der Nukleinsäuren verwendete Trägermaterial Nukleinsäuren aus extrem geringen Mengen, wie <0.5mg Gewebematerial; <0.5µl Blut oder Blutspuren auf Kleidungsstücken; <5µl Speichel; <10³ Zellen an unterschiedlichsten biologischen Ausgangsmaterialien isoliert werden.

11. Verfahren nach Anspruch 1 bis 10, dadurch gekennzeichnet, daß es zur schnellen Isolierung von genomischer Desoxyribonukleinsäure und zellulärer Gesamt- Ribonukleinsäure eingesetzt wird.

12. Verfahren nach Anspruch 1 bis 8, dadurch gekennzeichnet, daß es zur Isolierung und Reinigung von PCR-Produkten oder PCR-Fragmenten und Isolierung von DNS-Fragmenten aus wäßrigen Lösungen, TAE-oder TBE-Agarosegelen in einem breiten Molekularspektrum eingesetzt wird.

13. Verfahren nach Anspruch 1 bis 11, dadurch gekennzeichnet, daß zur Isolierung von Ribonukleinsäure die Lyse des biologischen Ausgangsmaterials mit 10-molarem Lithiumchlorid durchgeführt wird und die Lyse und Bindung der Ribonukleinsäuren an den SiO₂-Träger im selben Reaktionsgefäß erfolgt.

14. Verfahren nach Anspruch 1 bis 13, dadurch gekennzeichnet, daß die Größe der zu isolierenden oder zu reinigenden Nukleinsäuren den Bereich von 50 Nukleotiden bis 60 000 Nukleotiden umfaßt.

## Claims

1. Process for isolating and purifying nucleic acids from extremely small quantities of, and very highly contaminated, starting materials of extremely varied natures by
a) lysis of materials containing nucleic acids with buffers containing chaotropic salts at high ionic strength
b) incubation with a highly disperse, nonporous, unstructured and homogeneous SiO₂ carriers, in which the SiO₂ particles have grain sizes of 7 - 40 nm with active specific surface of 50 - 300 m²/g,
c) separation of the nucleic acids fixed on the carrier from the lysate,
d) washing the nucleic acids fixed on the surface of the carrier with a washing buffer, and
d) elution of the nucleic acids from the carrier with a buffer with low salt concentration.

2. Process according to Claim 1, characterized in that guanidine hydrochloride, guanidine thiocyanate, lithium chloride, sodium iodide, potassium iodide, sodium perchlorate or lithium chloride/urea mixtures with ionic strengths > 4 M are used as the chaotropic salts.

3. Process according to Claims 1 and 2, characterized in that highly disperse, nonporous, nonstructured and homogeneous chemically pure SiO₂ with a grain size of 40 nm and a specific surface of 50 m²/g is used as the carrier material.

4. Process according to Claims 1 to 3, characterized in that the lysis of the starting material containing the nucleic acids and the binding of the nucleic acids to the carrier particles is done as a 'single step' process in the same reaction vessel.

5. Process according to Claims 1 to 4, characterized in that the carrier with the bound nucleic acids is separated from the remainder of the lysate by a brief centrifugation step.

6. Process according to Claims 1 to 5, characterized in that the nucleic acids fixed on the carrier are washed with a washing buffer comprising 50 mM NaCl, 10 mM Tris-HCl and 1 mM EDTA and 70% ethanol.

7. Process according to Claims 1 to 6, characterized in that the nucleic acids fixed on the carrier are eluted with a buffer of low ionic strength, comprising 10 mM Tris-HCl, 0.1 mM EDTA, or other low-salt buffers, or are treated with DEPC at a temperature of 48-56 °C, preferably 52 °C.

8. Process according to Claims 1 to 7, characterized in that it is carried out as a batch process.

9. Process according to Claims 1 to 8, characterized in that the nucleic acid is derived from bacteria, cell cultures, intact or frozen tissue samples, tissue slices, sperm, body fluids, stool samples, viruses, plant cells, yeast cells, dried biological museum preparations, various forensic starting materials such as traces of blood on items of clothing, on tree bark, and the like; from hairs, saliva, bones or other biological sources, or from amplification reactions such as PCR or similar reactions, or that it is a labeled nucleic acid.

10. Process according to Claims 1 to 9, characterized in that, by use of the special carrier material used to bind the nucleic acids, nucleic acids are isolated from extremely minute quantities, such as < 0.5 mg tissue material; < 0.5 µl blood or blood traces on items of clothing; < 5 µl saliva; or < 10³ cells on extremely varied biological starting materials.

11. Process according to Claims 1 to 10, characterized in that it is used for rapid isolation of genomic deoxynucleic acid and cellular total ribonucleic acid.

12. Process according to Claims 1 to 8, characterized in that it is used to isolate and purify PCR products or PCR fragments and to isolate DNA fragments from aqueous solutions, or from TAE or TBE agarose gels over a broad molecular spectrum.

13. Process according to Claims 1 to 11, characterized in that, in isolation of ribonucleic acid, the biological starting material is lysed with 10 molar lithium chloride and the lysis and binding of the ribonucleic acids to the SiO₂ carrier are done in the same reaction vessel.

14. Process according to Claims 1 to 13, characterized in that the size of the nucleic acids being isolated or purified covers the range from 50 nucleotides to 60,000 nucleotides.

## Revendications

1. Procédé d'isolement et de purification d'acides nucléiques à partir de très petites quantités de substances de départ très diverses et très impures, par :
a) lyse des substances contenant des acides nucléiques avec des tampons contenant des sels chaotropes de forte concentration ionique,
b) incubation avec un support en SiO₂ fortement dispersé, non poreux, non structuré et homogène, les particules de SiO₂ ayant une taille de grains de 7 à 40 nm pour une surface active de 50 à 300 m²/g,
c) séparation des acides nucléiques fixés sur les supports et du lysat,
d) lavage des acides nucléiques fixés à la surface du support avec un tampon de lavage et
e) détachement des acides nucléiques du support avec un tampon de faible salinité.

2. Procédé selon la revendication 1, caractérisé en ce que l'agent chaotrope utilisé peut faire partie des sels suivants : chlorhydrate de guanidine, thiocyanate de guanidine, chlorure de lithium, iodure de sodium, iodure de potassium, perchlorate de sodium ou des mélanges de chlorure de lithium et d'urée d'une concentration ionique supérieure à 4 M.

3. Procédé selon les revendications 1 et 2, caractérisé en ce que le matériau de support est du SiO₂ fortement dispersé, non poreux, non structuré, homogène et chimiquement pur, ayant une taille de grains de 40 nm pour une surface active de 50 m²/g.

4. Procédé selon les revendications 1 à 3, caractérisé en ce que la lyse de la substance de départ contenant les acides nucléiques et la liaison des acides nucléiques sur les particules de support est réalisée en un seul temps dans le même récipient de réaction.

5. Procédé selon les revendications 1 à 4, caractérisé en ce que le support auquel sont liés les acides nucléiques est séparé du reste du lysat par une étape de centrifugation de courte durée.

6. Procédé selon les revendications 1 à 5, caractérisé en ce que les acides nucléiques fixés au support sont lavés avec un tampon de lavage composé de 50 mM de NaCl, 10 mM de tris-HCl et 1 mM d'EDTA et d'éthanol à 70 %.

7. Procédé selon les revendications 1 à 6, caractérisé en ce que les acides nucléiques fixés au support sont élués avec un tampon de faible densité ionique, composé de 10 mM de tris-HCl, 0,1 mM d'EDTA, d'autres tampons faiblement salins ou traités au DEPC, à une température de 48 à 56 °C, de préférence 52 °C.

8. Procédé selon les revendications 1 à 7, caractérisé en ce qu'il est réalisé selon le mode d'un procédé par lots.

9. Procédé selon les revendications 1 à 8, caractérisé en ce que les acides nucléiques proviennent de bactéries, de cultures cellulaires, d'échantillons de tissus intacts ou congelés, de coupes de tissus, de spermatozoïdes, de fluides corporels, d'échantillons de selles, de virus, de cellules végétales, de cellules de levures, de préparations biologiques sèches provenant de musées, de divers matériaux étudiés en médecine légale tels que des taches de sang sur des vêtements, de l'écorce, etc., de cheveux, de salive, d'os ou d'autres sources biologiques, ou de réactions d'amplification telles que la PCR ou des réactions similaires, ou sont des acides nucléiques marqués.

10. Procédé selon les revendications 1 à 9, caractérisé en ce que le matériau de support spécialement utilisé pour lier les acides nucléiques permet d'isoler des acides nucléiques dans des quantités extrêmement faibles, comme moins de 0,5 mg de tissus, moins de 0,5 µl de sang ou des traces de sang sur un vêtement, moins de 5 µl de salive, moins de 10³ cellules dans les substances biologiques de départ les plus diverses.

11. Procédé selon les revendications 1 à 10, caractérisé en ce qu'il est utilisé pour l'isolement d'acide désoxyribonucléique génomique et d'acide ribonucléique cellulaire.

12. Procédé selon les revendications 1 à 8, caractérisé en ce qu'il est utilisé pour isoler et purifier des produits de PCR ou des fragments de PCR et pour l'isolement de fragments d'ADN à partir de solutions aqueuses, de gels au TAE ou TAE-agarose dans un large spectre moléculaire.

13. Procédé selon les revendications 1 à 11, caractérisé en ce que pour l'isolement de l'acide ribonucléique, la lyse de la substance biologique de départ est réalisée avec du chlorure de lithium 10-molaire et la lyse et la liaison des acides ribonucléiques sur le support en SiO₂ sont réalisées dans le même récipient de réaction.

14. Procédé selon les revendications 1 à 13, caractérisé en ce que la taille des acides nucléiques à isoler ou à purifier peut aller de 50 à 60 000 nucléotides.
